(19) Europäisches Patentamt
European Patent Office
Office européen des brevets

(11) EP 1 490 053 B1

(12) **EUROPEAN PATENT SPECIFICATION**

(45) Date of publication and mention of the grant of the patent:
**09.01.2008 Bulletin 2008/02**

(21) Application number: **03714888.9**

(22) Date of filing: **17.03.2003**

(51) Int Cl.:
***A61K 31/40*** (2006.01) ***A61K 41/00*** (2006.01)
***A61P 35/00*** (2006.01)

(86) International application number:
**PCT/EP2003/003192**

(87) International publication number:
**WO 2003/082267 (09.10.2003 Gazette 2003/41)**

(54) **COMBINED THERAPY AGAINST TUMORS COMPRISING SUBSTITUTED ACRYLOYL DISTAMYCIN DERIVATIVES AND RADIOTHERAPY**

KOMBINIERTE TUMORTHERAPIE AUF DER BASIS VON DISTAMYCIN-ACRYLOYL DERIVATEN UND RADIOTHERAPIE

THERAPIE COMBINEE CONTRE DES TUMEURS CONTENANT DES DERIVES SUBSTITUES D'ACRYLOYL DISTAMYCINE ET RADIOTHERAPIE

(84) Designated Contracting States:
**AT BE BG CH CY CZ DE DK EE ES FI FR GB GR HU IE IT LI LU MC NL PT RO SE SI SK TR**

(30) Priority: **02.04.2002 EP 02076240**

(43) Date of publication of application:
**29.12.2004 Bulletin 2004/53**

(73) Proprietor: **NERVIANO MEDICAL SCIENCES S.r.l.**
**20014 Nerviano MI (IT)**

(72) Inventors:
- **GERONI, Maria, Cristina, Rosa**
  **I-20149 Milan (IT)**
- **TURSI, Jennifer, Margaret**
  **I-20146 Milan (IT)**
- **FOWST, Camilla**
  **I-20153 Milan (IT)**

(74) Representative: **Mazzini, Giuseppe et al**
**Nerviano Medical Sciences S.r.l.**
**Patent Department**
**Viale Pasteur, 10**
**20014 Nerviano (Milano) (IT)**

(56) References cited:
**WO-A-01/97790** **WO-A-98/04524**

- **MARCHINI S ET AL: "Development of distamycin-related DNA binding anticancer drugs." EXPERT OPINION ON INVESTIGATIONAL DRUGS. ENGLAND SEP 2001, vol. 10, no. 9, September 2001 (2001-09), pages 1703-1714, XP002243510 ISSN: 1354-3784**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 490 053 B1

## Description

The present invention relates to the field of cancer treatment and provides an antitumor therapy comprising the combined use of a substituted acryloyl distamycin derivative, more particularly a α-bromo- or α-chloro-acryloyl-distamycin derivative, with radiotherapy.

The treatment of tumours with ionising radiation, also referred to as radiotherapy, is extensively used in cancer therapy as it provides destruction of tumour cells together with inhibition of tumour cell growth, presumably though DNA damage.

Some therapeutic compounds, which are known as being cytotoxic per se, hence susceptible of being used in the therapy of cancer, are also endowed with radiosensitisation activity as they are capable of inducing DNA radiation damage in response to ionizing radiation.

So far, the possibility of combining both cytotoxic agents, e.g. a given radiosensitiser and radiotherapy, with the expectation of getting a supra-additive antitumor effect in comparison to the single cytotoxics alone, is of utmost importance in cancer therapy. Among the several compounds endowed with antitumor activity and also known as possessing radiosensitisation activity see, for instance, cisplatin, gemcitabine, navelbine, tomudex, nicotinamide, paclitaxel, docetaxel, simvastatin and topotecan.

In addition, the use of halogenated DNA ligands as possible radiosensitisers, also including some distamycin derivatives, were disclosed by R. Martin et al. in the international patent application WO 90/12321.

For a general reference to distamycin, an antibiotic substance with antiviral and antiprotozoal activity, as well as to the several derivatives thereof which are known as cytotoxic agents see, for instance, Nature 203: 1064 (1964); J. Med. Chem. 32: 774-778 (1989); and the international patent applications WO 90/11277, WO 98/21202, WO 99/50265, WO 99/50266 and WO 01/40181, all in the name of the applicant itself.

Among the several distamycin derivatives being disclosed so far, a class of α-bromo- or α-chloro-acryloyl-distamycins, as per the aforementioned international patent application WO 98/04524, were found to possess a significant antineoplastic activity.

We have now found that these same compounds are also unexpectedly endowed with a remarkable radiosensitisation activity which render their use, in combination with radiotherapy, particularly advantageous in cancer therapy.

Such compounds can be used in the preparation of a medicament having radiosensitisation activity.

In the present description, unless otherwise specified, with the term "radiosensitisation activity" it is intended the aforementioned capability of a compound, or medicament thereof, to act as a radiosensitiser. With the term "radiosensitiser", in its turn, we refer to a compound or medicament which is capable of increasing or otherwise improving tumor cells destruction in response to ionizing radiation.

Finally, the term "ionizing radiation" is the one conventionally adopted in the therapeutic field of cancer treatment and includes photons having enough energy for bonds ionization such as, for instance, α-, β- and γ-rays from radioactive nuclei as well as x-rays.

The α-bromo- or α-chloro-acryloyl-distamycin derivative which is used according to the present invention is a compound of formula (I) below

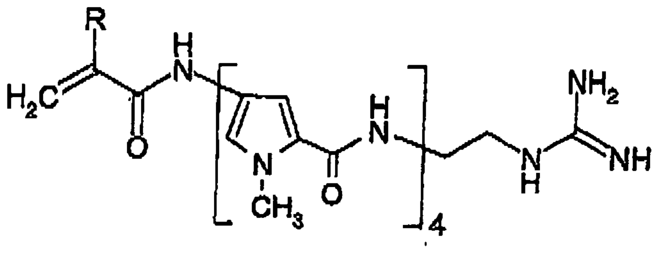

(I)

wherein R is a bromine or chlorine atom, more preferably bromine, or a pharmaceutically acceptable salt thereof.

Pharmaceutically acceptable salts of the compounds of formula (I) are the salts with pharmaceutically acceptable inorganic or organo acids such as, for instance, hydrochloric, hydrobromic, sulfuric, nitric, acetic, propionic, succinic, malonic, citric, tartaric, methanesulfonic, p-toluenesulfonic acid and the like; the hydrochloride salt being the preferred one.

Even more preferably, the acryloyl-distamycin derivative for use as radiosensitiser is the compound N-[5-[[[5-[[[2-[(aminoiminomethyl)amino]ethyl]amino]carbonyl]-1-methyl-1H-pyrrol-3-yl]amino]carbonyl]-1-methyl-1H-pyrrol-3-yl]-4-[[[4-[(2-bromo-1-oxo-2-propenyl)amino]-1-methyl-1H-pyrrol-2-yl]carbonyl]amino]-1-methyl-1H-pyrrole-2-carboxamide hydrochloride (internal code PNU 166196A).

The combined therapy is suitable for the treatment of various tumor forms such as, for instance, breast, ovary,

lung, colon (including rectus), kidney, stomach, pancreas, liver, head and neck, esophagus, uterus (including body and cervix), vagina, melanoma and non malanoma skin cancer, as well as sarcomas.

From all of the above and unless otherwise specified, it is clear to the skilled person that the α-bromo- or α-chloro-acryloyl-distamycin derivative may be administered to mammals, including humans, through the usual routes, for example parenterally, e.g. by intravenous injection or infusion.

The dosage will depend from several factors, iso including the selected schedule of administration which may comprise repeated doses, for instance once a day, once a week, twice a week, and the like, as the case may be.

As a non limiting example, suitable dosages may range from about 0.05 $mg/m^2$ to about 10 $mg/m^2$.

For any indication concerning suitable pharmaceutical forms for administering the acryloyl-distamycin derivatives in re, hence including any pharmaceutically acceptable excipient, see the aforementioned international patent application WO 98/04524.

The present invention thus provides the use of a α-bromo- or α-chloro-acryloyl-distamycin-derivative of formula (I) for manufacturing a medicament for treating a mammal, including humans, suffering from a neoplastic disease state, by administration to said mammal of said α-bromo- or α-chloro-acryloyl-distamycin derivative in combination with radiotherapy, in amounts and according to a schedule treatment effective to produce a synergistic antineoplastic effect.

By the term "synergistic" effect, as used herein, it is meant the inhibition of the growth tumour, preferably the complete regression of the tumor, by administering an effective amount of the above acryloyl distamycin derivative and the ionizing radiation to mammals, including humans. By the term "administered " or "administering", as used herein, it is meant parenteral (e.g. intravenous) administration.

As far as the schedule treatment is concerned, exposure to radiotherapy may either occur simultaneously whilst administering the medicament comprising the α-bromo- or α-chloro-acryloyl-distamycin derivative or, alternatively, sequentially in any order.

Preferably, the schedule treatment first comprises administering the drug to the patient which only subsequently is subjected to radiotherapy exposure.

According to the present invention, the acryloyl on derivative may be also administered with additional antitumor agents such as, for instance, topoisomerase I or II inhibitors, e.g. CPT-11, topotecan, 9-amino-camptothecin, 9-nitro-camptothecin, 10,11-methylenedioxy-camptothecin, doxorubicin, daunorubicin, epirubicin, nemorubicin, idarubicin, etoposide, teniposide, mitoxanthrone, losoxantrone, amsacrine, actinomycin D; alkylating agents, e.g. melphalan, chlorambucil, mechlorethamine, cyclophosphamide, ifosfamide, busulfan, carmustine, lormustine, semustine, fotemustine, decarbazine, temozolide, thitepa, mitomycin C, cisplatin, carboplatin, oxaliplatin, nedaplatin, lobaplatin; antimicrotubule agents, e.g. paclitaxel, docetaxel, vincristine, vinblastine, vindesine, vinorelbine, estramustine; antimetabolites, e.g. metotrexate, trimetrexate, tomudex, 5-FU, floxuridine, ftorafur, capecitabine, cytarabine, azacitidine, gemcitabine; protein kinase inhibitors, e.g. STI571 (Gleevec), ZD-1839 (Iressa), OSI-774 (Tarceva), SU 5416 (Semaxanib), SU 6668, SU 11248; retinoid derivatives, e.g. cis-retinoic acids, trans-retinoic acids; cyclooxygenase inhibitors such as COX-2 inhibitors, e.g. celecoxib, rofecoxib, parecoxib, valdecoxib; hormonal agents, e.g. exemestane, formestane, atamestane, letrozole, fadrozole, anastrozole.

Preferably, the treatment of said mammal comprises the use of the α-bromo- or α-chloro-acryloyl-distamycin derivative with radiotherapy and the administration of a platinum alkylating agent, more preferably cisplatin,

PHARMACOLOGY

The remarkable radiosensitisation effect exerted by the α-bromo- or α-chloro-acryloyl-distamycin derivatives, in particular the compounds of formula (I), is shown according to in vitro clonogenic assays on SQ20B (radiation-resistant human squamous cell carcinoma of the larynx) and A431 (human vulval carcinoma) cell lines. In this respect, two different schedule treatments were evaluated either comprising simultaneous exposure to the tested compound of formula (I) and to radiation, or sequential exposure to both these cytotoxic agents in any order, that is drug/radiation or radiation/drug (see details below). As control, the effect of cisplatin in combination with radiotherapy has been tested in the same operative conditions.

To define a Sensitization Ratio (SR), the clonogenic survival of cells being treated with a combination or irradiation and drug exposure ($S_{X+D}$) was compared with the product of survival for drug alone ($S_D$) and irradiation alone ($S_X$), as follows

$$SR = S_{X+D} / S_D \cdot S_X$$

From the above, it is clear to the skilled person that if both radiation and drug exerted their cytotoxic effect independently from each other, SR values would be close to 1 whereas, on the contrary, a radiosensitisation effect

indicating a synergism between ionizing radiation and drug is characterized by SR values lower than 1 (SR < 1).

Analysis of the obtained results in any of the experiments being carried out clearly indicate that the tested compound of formula (I) exerts a remarkable and statistically significant radiosensitising effect.

In particular, whilst sensitization is substantially comparable to that of cisplatin on SQ20B cell line, it is unexpectedly and significantly superior than that of cisplatin on A431 cell line, hence indicating a possible widest range of applications for the compounds of formula (I), in combination with radiotherapy.

In addition to the above, we unexpectedly found that the radiosensitisation effect of the compound of formula (I) could be even increased, to a statistically significant extent, when drug exposure occurred before irradiation treatment, according to one of the sequential schedule treatments.

To better illustrate the present invention, without posing any limitation to it, the following examples are now given.

**Example 1**

**Radiosensitisation activity of PNU 166196A in comparison to cisplatin**

For both compounds PNU 166196A and cisplatin, exposures were simultaneous to ionizing radiation in both SQ20B and A431 cell lines. The schedule consisted of 2 h drug treatment with a period of irradiation (10 minutes) starting at the beginning of the 2nd hour of treatment.

Four data sets for each of PNU 166196A and cisplatin, in each cell line were obtained (see table 1) comprising duplicates of two different drug concentration chosen to yield cytotoxicity values corresponding to 80% ($C_{80}$) and 20% ($C_{20}$) survival for treatment with the drug alone.

**Table 1 - Sensitisation ratio of PNU 166196A and cisplatin in combination with radiotherapy**

| Cell line | Drug | Drug Concentration[(a)] | Sensitisazion Ratio SR[(b)] | |
|---|---|---|---|---|
| | | | Values for each culture | Mean of duplicates |
| SQ20B | PNU 166196A | 50 ($C_{80}$) [(c)] | 0.85 | **$C_{80}$ 0.85** |
| | | 350 ($C_{20}$) [(d)] | 0.46 | **$C_{20}$ 0.50** |
| | | 50 ($C_{80}$) | 0.84 | |
| | | 350 ($C_{20}$) | 0.55 | p=0.017 |
| | cisplatin | 0.4 ($C_{80}$) | 0.78 | **$C_{80}$ 0.80** |
| | | 6.5 ($C_{20}$) | 0.72 | **$C_{20}$ 0.77** |
| | | 0.4 ($C_{80}$) | 0.82 | |
| | | 6.5 ($C_{20}$) | 0.81 | p=0.034 |
| A431 | PNU 166196A | 20 ($C_{80}$) | 0.65 | **$C_{80}$ 0.62** |
| | | 90 ($C_{20}$) | 0.33 | **$C_{20}$ 0.37** |
| | | 20 ($C_{80}$) | 0.59 | |
| | | 90 ($C_{20}$) | 0.40 | p=0.029 |
| | cisplatin | 0.8 ($C_{80}$) | 1.02 | **$C_{80}$ 0.96** |
| | | 5.0 ($C_{20}$) | 1.08 | **$C_{20}$ 0.97** |
| | | 0.8 ($C_{80}$) | 0.84 | |
| | | 5.0 ($C_{20}$) | 0.86 | p=0.37 |

(a) Expressed as ng/ml for PNU 166196A and μM for cisplatin;
(b) SR values lower than 1 (SR < 1) indicate radiosensitisation;
(c) $C_{80}$ drug concentration corresponding to 80% cell survival
(d) $C_{20}$ drug concentration corresponding to 20% cell survival

From the above, SR for PNU 166196A is lower than 1 in both cell lines being investigated; on A431, SR for PNU 166196 is markedly lower than that of cisplatin, hence indicating a superior radiosensitisation effect.

**Example 2**

**Radiosensitisation activity of PNU 166196A under sequential schedule treatment**

PNU 166196A was tested in both SQ20B and A431 cell lines, according to two sequential schedule treatments comprising: 2 h drug treatment ending 60 minutes before irradiation (drug-before schedule) and 2 h drug treatment starting 40 minutes after irradiation (drug-after schedule), the irradiation period being of 10 minutes, in each case. For each cell line, PNU 166196A was tested at the highest concentration (see table 2) to yield cytotoxicity values corresponding to 20% ($C_{20}$) survival for treatment with the drug alone.

**Table 2 - Effect of the sequence of treatment on the sensitisation ratio of PNU 166196 in combination with radiotherapy**

| Cell line | Drug Concentration (ng/ml) | Sensitisazion Ratio SR [(a)] | |
|---|---|---|---|
| | | Drug-before [(b)] | Drug-after [(c)] |
| SQ20B | 350 ($C_{20}$) [(d)] | 0.15 | 0.62 |
| | 350 ($C_{20}$) | 0.47 | 0.73 |
| A431 | 90 ($C_{20}$) | 0.11 | 0.87 |
| | 90 ($C_{20}$) | 0.13 | 0.43 |
| Paired t-test | | P=0.043 | P=0.074 |

[(a)] SR values lower than 1 (SR < 1) indicate radiosensitisation;
[(b)] 2 h exposure to PNU 16196A before irradiation;
[(c)] 2 h exposure to PNU 16196A after irradiation;
[(d)] $C_{20}$ drug concentration corresponding to 20% cell survival

From the above, even if SR values are lower than 1 in both cell lines and according to both schedules, the radiosensitisation activity of PNU 166196A is significantly higher (SR<<1) when the treatment with the compound is carried out before irradiation.

## Claims

1. Use of a α-bromo or α-chloro-acryloyl-distamycin derivative for manufacturing a medicament for treating a mammal, including humans, suffering from a neoplastic disease state, wherein the medicaments is to be administered to said mammal in combination with radiotherapy in amounts and according to a schedule treatment effective to produce a synergistic antineoplastic effect, wherein the α-bromo or α-chloro-acryloyl-distamycin derivative is of the formula (I)

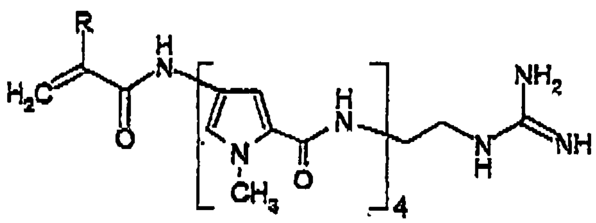

(I)

wherein R is a bromine or chlorine atom, or a pharmaceutically acceptable salt thereof.

2. The use according to claim 1 wherein, within formula (I), R is a bromine atom.

3. The use according to claim 1 wherein the acryloyl-distamycin derivative is the compound N-[5-[[[5-[[[2-[(aminoiminomethyl)amino]ethyl]amino]carbonyl]-1-methyl-1H-pyrrol-3-yl]amino]carbonyl]-1-methyl-1H-pyrrol-3-yl]-4-[[[4-[(2-bromo-1-oxo-2-propenyl)amino]-1-methyl-1H-pyrrol-2-yl]carbonyl]amino]-1-methyl-1H-pyrrole-2-carboxamide hydrochloride.

4. The use according to claim 1 wherein the neoplastic disease state is selected from the group consisting of breast, ovary, lung, colon (including rectus), kidney, stomach, pancreas, liver, head and neck, esophagus, uterus (including body and cervix), vagina, melanoma and non melanoma skin cancer, as well as sarcomas.

5. The use according to claim 1 wherein exposure to radiotherapy occurs either simultaneously whilst administering the medicament of claim 1, or sequentially, in any order.

6. The use according to claim 1 wherein treating said mammal first comprises administering the α-bromo or α-chloro-acryloyl-distamycin derivative to the patient and subsequently subjecting the said patient to radiotherapy.

7. The use according to claim 1 wherein treating said mammal optionally further comprises the administration of an additional antitumor agent, either separately, simultaneously or sequentially, in any order.

8. The use according to claim 7 wherein the additional antitumor agent is selected from the group consisting of topoisomerase I or II inhibitors, alkylating agents, antimicrotubule agents, antimetabolites, protein kinase inhibitors, retinoid derivatives, cyclooxygenase inhibitors and hormonal agents.

9. The use according to claim 8 wherein the additional antitumor agent is cisplatin.

**Patentansprüche**

1. Verwendung von einem α-Brom- oder α-Chlor-Acryloyl-Distamycin Derivat zur Herstellung von einem Medikament zur Behandlung eines Säugers, einschließlich der Menschen, der an einem neoplastischen Krankheitszustand leidet, wobei das Medikament an den Säuger in Kombination mit Strahlentherapie zu verabreichen ist, in Mengen und gemäß einem Behandlungsschema, das leistungsfähig ist, eine synergistische antineoplastische Wirkung hervorzurufen; wobei das α-Brom- oder α-Chlor-Acryloyl-Distamycin Derivat von der Formel (I) ist,

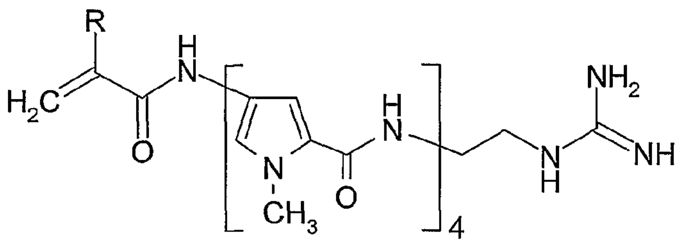

(I)

wobei R ein Brom- oder Chloratom oder ein pharmazeutisch unbedenkliches Salz daraus bedeutet.

2. Verwendung nach Anspruch 1, wobei R innerhalb der Formel (I) ein Bromatom ist.

3. Verwendung nach Anspruch 1, wobei das Acryloyl-Distamycin Derivat die Verbindung N-[5-[[[5-[[[2-(Aminoiminomethyl)amino]ethyl]amino]carbonyl]-1-methyl-1H-pyrrol-3-yl]amino]carbonyl]-1-methyl-1H-pyrrol-3-yl]-4-[[[4-[(2-brom-1-oxo-2-propenyl)amino]-1-methyl-1H-pyrrol-2-yl]carbonyl]amino]-1-methyl-1H-pyrrol-2-carboxamid Hydrochlorid ist.

4. Verwendung nach Anspruch 1, wobei der neoplastische Krankheitszustand ausgewählt ist aus der Gruppe der Tumore von Brust, Eierstock, Lunge, Darm (einschließlich Mastdarm), Niere, Magen, Bauchspeicheldrüse, Leber, Kopf und Hals, Speiseröhre, Gebärmutter (einschließlich Gebärmutterkörper und Gebärmutterhals) und Scheide, sowie Melanom- und Nicht-Melanom-Hautkrebs und Sarkomen.

5. Verwendung nach Anspruch 1, wobei die Exposition zu der Strahlentherapie entweder gleichzeitig während der Verabreichung des Medikamentes nach Anspruch 1 oder aufeinanderfolgend in beliebiger Reihenfolge erfolgt.

6. Verwendung nach Anspruch 1, wobei das Behandeln des Säugers zuerst das Verabreichen des α-Brom- oder α-Chlor-Acryloyl-Distamycin Derivates an den Patienten umfasst und anschließend der Patient einer Strahlentherapie unterzogen wird.

7. Verwendung nach Anspruch 1, wobei das Behandeln des Säugers ferner wahlweise das Verabreichen von einem zusätzlichen Antitumormittel, entweder getrennt, gleichzeitig oder aufeinanderfolgend, in beliebiger Reihenfolge umfasst.

8. Verwendung nach Anspruch 7, wobei das zusätzliche Antitumormittel ausgewählt ist aus der Gruppe bestehend aus Topoisomerase I- oder II-Inhibitoren, alkylierenden Substanzen, antimikrotubulären Substanzen, Antimetaboliten, Proteinkinase-Inhibitoren, Retinoid-Derivaten, Cyclooxygenase-Inhibitoren und hormonell wirksamen Substanzen.

9. Verwendung nach Anspruch 8, wobei das zusätzliche Antitumormittel Cisplatin ist.

**Revendications**

1. Utilisation d'un dérivé de distamycine d'α-bromo ou d'α-chloroacryloyle pour fabriquer un médicament pour traiter un mammifère, notamment des humains, souffrant d'une maladie néoplasique, dans laquelle le médicament doit être administré audit mammifère en combinaison avec une radiothérapie dans des quantités et selon un traitement programmé efficace pour produire un effet antinéoplasique synergique, dans laquelle le dérivé de distamycine d'α-bromo ou d'α-chloroacryloyle est de formule (I)

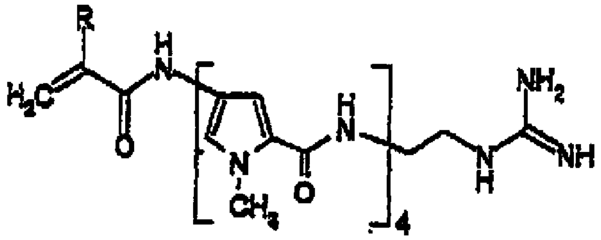

(I)

dans laquelle R est un atome de brome ou de chlore, ou un sel pharmaceutiquement acceptable de celui-ci.

2. Utilisation selon la revendication 1 dans laquelle, dans la formule (I), R est un atome de brome.

3. Utilisation selon la revendication 1 dans laquelle le dérivé de distamycine d'acryloyle est le composé chlorhydrate de N-[5-[[[5-[[[2-[(aminoiminométhyl)amino]éthyl]amino]carbonyl]-1-méthyl-1H-pyrrol-3-yl]amino]carbonyl]-1-méthyl-1H-pyrrol-3-yl]-4-[[[4-[(2-bromo-1-oxo-2-propényl)amino]-1-méthyl-1H-pyrrol-2-yl]carbonyl]amino]-1-méthyl-1H-pyrrole-2-carboxamide.

4. Utilisation selon la revendication 1 dans laquelle la maladie néoplasique est choisie dans le groupe constitué par le cancer du sein, de l'ovaire, du poumon, du côlon (notamment du rectum), du rein, de l'estomac, du pancréas, du foie, de la tête et du cou, de l'oesophage, de l'utérus (notamment du corps et du col), du vagin, le cancer de la peau avec présence de mélanome et avec absence de mélanome, ainsi que les sarcomes.

5. Utilisation selon la revendication 1 dans laquelle l'exposition à la radiothérapie s'effectue soit simultanément tout en administrant le médicament de la revendication 1, soit séquentiellement, dans un ordre quelconque.

6. Utilisation selon la revendication 1 dans laquelle le traitement dudit mammifère comprend d'abord l'administration du dérivé de distamycine d'α-bromo ou d'α-chloroacryloyle au patient puis la soumission dudit patient à une radiothérapie.

7. Utilisation selon la revendication 1 dans laquelle le traitement dudit mammifère comprend en outre facultativement l'administration d'un agent antitumoral supplémentaire, séparément, simultanément ou séquentiellement, dans un ordre quelconque.

8. Utilisation selon la revendication 7 dans laquelle l'agent antitumoral supplémentaire est choisi dans le groupe constitué par les inhibiteurs de la topoisomérase I ou II, les agents alkylants, les agents antimicrotubules, les antimétabolites, les inhibiteurs de la protéine kinase, les dérivés rétinoïdes, les inhibiteurs de la cyclooxygénase et les agents hormonaux.

**9.** Utilisation selon la revendication 8 dans laquelle l'agent antitumoral supplémentaire est la cisplatine.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9012321 A, R. Martin
- WO 9011277 A
- WO 9821202 A
- WO 9950265 A
- WO 9950266 A
- WO 0140181 A
- WO 9804524 A


### Non-patent literature cited in the description


- *Nature,* 1964, vol. 203, 1064
- *J. Med. Chem.,* 1989, vol. 32, 774-778